# EUROPÄISCHE PATENTANMELDUNG

(11) **EP 3 536 238 A1**
(43) Veröffentlichungstag der Anmeldung: **11.09.2019**
(21) Anmeldenummer: 18160220.2
(22) Anmeldetag: 06.03.2018
(51) Int. Cl.: A61B 5/055, A61B 5/107, A61B 5/08, A61B 5/0402, G01R 33/20, G16H 10/60, A61B 90/00, A61B 6/03, A61B 6/04

(54) **VERFAHREN ZU EINER UNTERSTÜTZUNG EINER VORBEREITUNG EINES PATIENTEN FÜR EINE MEDIZINISCHE BILDGEBUNGSUNTERSUCHUNG**

(71) Anmelder: Siemens Healthcare GmbH, 91052 Erlangen (DE)
(72) Erfinder: Zeller, Mario, 91054 Erlangen (DE)

(57) **Zusammenfassung**

Die Erfindung geht aus von einem Verfahren zu einer Unterstützung einer Vorbereitung eines Patienten für eine medizinische Bildgebungsuntersuchung, insbesondere eine Magnetresonanzuntersuchung, mit den folgenden Schritten:
- Erfassen von Patientendaten eines Patienten mittels einer Erfassungseinheit,
- Berechnen einer Positionsinformation eines Objekts mittels einer Berechnungseinheit, wobei das Berechnen der Positionsinformation des Objekts anhand der Patientendaten und/oder anhand einer Untersuchungsinformation und/oder anhand von Daten von Zubehöreinheiten erfolgt, und
- Projizieren der Positionsinformation des Objekts mittels einer Projektionseinheit.

## Beschreibung

Die vorliegende Erfindung betrifft ein Verfahren zu einer Unterstützung einer Vorbereitung eines Patienten für eine medizinische Bildgebungsuntersuchung. Des Weiteren betrifft die vorliegende Erfindung auch eine medizinische Bildgebungsvorrichtung, die zu einer Ausführung des Verfahrens zu einer Unterstützung einer Vorbereitung eines Patienten für eine medizinische Bildgebungsuntersuchung ausgelegt ist.

Ein wesentlicher Faktor für eine Bildqualität bei medizinischen Bildgebungsuntersuchungen, wie beispielsweise bei Magnetresonanzuntersuchungen, ist eine ideale und/oder optimale Positionierung von möglichen Zubehöreinheiten, wie beispielsweise lokale Hochfrequenzantenneneinheiten, am Patienten. Die Positionierung der Zubehöreinheiten wird von einem medizinischen Bedienpersonal durchgeführt, so dass die optimale Positionierung der Zubehöreinheiten oft von einem Erfahrungswert des medizinischen Bedienpersonals abhängig ist. Wird die Positionierung von Zubehöreinheiten von einem unerfahrenen und/oder ungeübten medizinischen Bedienpersonal durchgeführt, kann dies zu Problemen bei der Positionierung führen und damit auch zu Problemen bei der Bildqualität führen.

Verfahren zur Positionierung sind aus DE 10 2016 209 297 A1 sowie die DE 10 2016 208 018 A1 bekannt.

Der vorliegenden Erfindung liegt insbesondere die Aufgabe zugrunde, eine Vorbereitung eines Patienten für eine medizinische Bildgebungsuntersuchung, insbesondere eine Magnetresonanzuntersuchung, für ein medizinisches Bedienpersonal zu vereinfachen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einer Unterstützung einer Vorbereitung eines Patienten für eine medizinische Bildgebungsuntersuchung, insbesondere eine Magnetresonanzuntersuchung, mit den folgenden Schritten:
- Erfassen von Patientendaten eines Patienten mittels einer Erfassungseinheit,
- Berechnen einer Positionsinformation eines Objekts mittels einer Berechnungseinheit, wobei das Berechnen der Positionsinformation des Objekts anhand der Patientendaten und/oder anhand einer Untersuchungsinformation und/oder anhand von Daten von Zubehöreinheiten erfolgt, und
- Projizieren der Positionsinformation des Objekts mittels einer Projektionseinheit.

Vor einem Start einer medizinischen Bildgebungsuntersuchung muss zunächst der zu untersuchende Patient von einem medizinischen Bedienpersonal für diese medizinische Bildgebungsuntersuchung vorbereitet werden. Zu Vorbereitung des Patienten zählt beispielsweise ein Positionieren des Patienten auf einer Patientenlagerungsvorrichtung. Die Positionierung des Patienten auf der Patientenlagerungsvorrichtung ist zudem abhängig von einem zu untersuchenden Bereich des Patienten. Beispielsweise muss der Patient für eine Kopfuntersuchung derart auf der Patientenlagerungsvorrichtung platziert werden, dass der Patient mit dem Kopf voran in einen Patientenaufnahmebereich der medizinischen Bildgebungsvorrichtung eingefahren wird. Für eine Fußuntersuchung und/oder eine Knieuntersuchung dagegen muss der Patient derart auf der Patientenlagerungsvorrichtung platziert werden, dass der Patient mit den Füssen voran in einen Patientenaufnahmebereich der medizinischen Bildgebungsvorrichtung eingefahren wird.

Neben einer Positionierung des Patienten werden zur Vorbereitung des Patienten für die medizinische Bildgebungsuntersuchung auch noch, sofern erforderlich, Zubehöreinheiten an dem Patienten angebracht. Derartige Zubehöreinheiten können beispielsweise eine lokale Hochfrequenzantenneneinheit für eine Magnetresonanzuntersuchung am Patienten sein. Zudem kann die Zubehöreinheit auch eine EKG-Einheit und/oder Atemsensoren und/oder weitere, dem Fachmann als sinnvoll erscheinende Zubehöreinheiten umfassen. Diese Zubehöreinheiten müssen von dem medizinischen Bedienpersonal korrekt, insbesondere mit der korrekten Orientierung an der korrekten Position, am Patienten positioniert werden.

Das Erfassen der Patientendaten erfolgt vorzugsweise selbsttätig und/oder automatisch mittels der Erfassungseinheit. Die Erfassungseinheit kann dabei an einer Scannereinheit der medizinischen Bildgebungsvorrichtung angeordnet sein oder auch an einer Wand oder Zimmerdecke eines Untersuchungsraums, innerhalb dessen die Scannereinheit der medizinischen Bildgebungsvorrichtung angeordnet ist. Vorzugsweise weist die Erfassungseinheit einen Erfassungsbereich auf, wobei der Erfassungsbereich einen Bereich umfasst, in dem der Patient für die medizinische Bildgebungsuntersuchung vorbereitet wird. Dieser Erfassungsbereich umfasst bevorzugt einen Bereich vor der Scannereinheit der medizinischen Bildgebungsvorrichtung, wobei dieser Bereich an eine Frontseite der Scannereinheit angrenzt.

Die Patientendaten umfassen bevorzugt eine Lage und/oder Orientierung und/oder eine Ausdehnung, insbesondere eine Größe und/oder eine Länge und/oder eine Breits und/oder eine Höhe, des Patienten. Alternativ oder zusätzlich können die Patientendaten auch eine Position des Patienten auf der Patientenlagerungsvorrichtung und/oder bezüglich der Patientenlagerungsvorrichtung umfassen. Zusätzlich können die Patientendaten auch nur einzelne Körperbereiche und/oder Körperregionen des Patienten umfassen, wie beispielsweise Abmessungen eines Arms oder des Kopfs oder des Oberkörpers usw. umfassen.

Die erfassten Patientendaten werden bevorzugt mittels einer Datenübertragungseinheit von der Erfassungseinheit an die Berechnungseinheit übertragen. Hierbei kann es vorgesehen sein, dass die Berechnungseinheit die erfassten Patientendaten auch aktiv von der Erfassungseinheit abruft. Die Datenübertragung zwischen der Berechnungseinheit und der Erfassungseinheit kann dabei kabelgebunden oder auch kabellos oder auch drahtlos erfolgen.

Das Berechnen der Positionsinformation eines Objekts erfolgt mittels der Berechnungseinheit. Vorzugsweise erfolgt das Berechnen der Positionsinformation des Objekts automatisch und/oder selbsttätig mittels der Berechnungseinheit. Die Berechnungseinheit weist hierzu einen Prozessor auf. Des Weiteren umfasst die Berechnungseinheit auch entsprechende Berechnungsprogramme und/oder Berechnungssoftware, die bei einem Ausführen ein Verfahren zu einem Unterstützen einer Vorbereitung eines Patienten für eine medizinische Bildgebungsuntersuchung ausführen. Die Berechnungsprogramme und/oder Berechnungssoftware können dabei innerhalb einer Speichereinheit der Berechnungseinheit und/oder der medizinischen Bildgebungsvorrichtung gespeichert sein. Zudem können die Berechnungsprogramme und/oder Berechnungssoftware auch in einem externen Speicher, wie beispielsweise einer Cloud, hinterlegt sein, wobei die Berechnungseinheit hierbei mittels eines Datennetzwerks auf die Berechnungsprogramme und/oder Berechnungssoftware zugreifen kann. Die Berechnungseinheit kann innerhalb einer Steuerungseinheit der medizinischen Bildgebungsvorrichtung integriert sein. Alternativ oder zusätzlich kann die Berechnungseinheit auch als selbstständige Einheit ausgebildet sein.

Die Untersuchungsinformation umfasst beispielsweise eine Untersuchungsregion und/oder einen zu untersuchenden Bereich des Patienten. Die Untersuchungsregion kann bereits in einer Patientendatenbank hinterlegt sein, auf die die Berechnungseinheit zugreifen kann. Beispielsweise kann die Untersuchungsregion bereits bei einer Patientenregistrierung erfasst werden und in der Patientendatenbank hinterlegt werden. Zudem ist es auch denkbar, dass die Untersuchungsregion während der Vorbereitung von dem medizinischen Bedienpersonal übermittelt wird, wie beispielsweise mittels einer manuellen Eingabe der Untersuchungsregion. Die Untersuchungsinformation kann beispielsweise auch ein Krankheitsbild des Patienten umfassen, anhand dessen eine Untersuchungsregion festgelegt wird. Das Festlegen der Untersuchungsregion anhand eines Krankheitsbilds kann auch automatisch und/oder selbsttätig mittels der Berechnungseinheit erfolgen. Beispielsweise kann bei einem Krankheitsbild "Kopfschmerzen des Patienten" anhand dessen die Untersuchungsregion zunächst auf den Kopf des Patienten festgelegt werden kann.

Die Daten der Zubehöreinheiten sind bevorzugt innerhalb einer Datenbank hinterlegt, wobei die Berechnungseinheit mittels des Datennetzwerkes auf die Daten der Zubehöreinheiten der Datenbank zugreifen kann. Die Daten der Zubehöreinheiten umfassen typischerweise eine Geometrie, insbesondere eine Länge und/oder eine Breite und/oder eine Höhe, der einzelnen Zubehöreinheiten. Alternativ oder zusätzlich können die Daten der Zubehöreinheiten auch eine Sollposition der einzelnen Zubehöreinheiten bezüglich der Patientenlagerungsvorrichtung und/oder bezüglich des Patienten umfassen. Zudem können die Daten der einzelnen Zubehöreinheiten auch einen Einsatzbereich, insbesondere für welche Art von Untersuchungen die einzelnen Zubehöreinheiten verwendet werden können, umfassen.

Die Positionsinformation des Objekts kann dabei eine Positionsinformation des Patienten umfassen. Beispielsweise kann hierdurch eine Position des Patienten für die anstehende medizinische Bildgebungsuntersuchung festgelegt werden und/oder auch bezüglich einer Zubehöreinheit überprüft und/oder korrigiert werden. Des Weiteren kann die Positionsinformation des Objekts auch eine Positionsinformation einer Zubehöreinheit umfassen. Beispielweise kann die Positionsinformation eine Sollposition einer Zubehöreinheit bezüglich des Patienten und/oder der Patientenlagerungsvorrichtung umfassen.

Die berechneten Positionsinformationen des Objekts werden bevorzugt mittels einer Datenübertragungseinheit von der Berechnungseinheit an die Projektionseinheit übertragen. Hierbei kann es auch vorgesehen sein, dass die Berechnungseinheit die berechneten Positionsinformationen des Objekts aktiv an die Projektionseinheit übermittelt und/oder sendet. Die Datenübertragung zwischen der Berechnungseinheit und der Projektionseinheit kann dabei kabelgebunden oder auch kabellos oder auch drahtlos erfolgen.

Das Projizieren der Positionsinformation des Objekts erfolgt bevorzugt mittels der Projektionseinheit. Die Projektionseinheit kann dabei an der Scannereinheit der medizinischen Bildgebungsvorrichtung angeordnet sein oder auch an einer Wand oder Zimmerdecke des Untersuchungsraums, innerhalb dessen die Scannereinheit der medizinischen Bildgebungsvorrichtung angeordnet ist. Vorzugsweise ist ein Projektionsbereich der Projektionseinheit auf den Patienten und/oder die Patientenlagerungsvorrichtung während einer Vorbereitung des Patienten für die medizinische Bildgebungsuntersuchung gerichtet. Dieser Projektionsbereich umfasst bevorzugt einen Bereich vor der Scannereinheit der medizinischen Bildgebungsvorrichtung, wobei dieser Bereich an eine Frontseite der Scannereinheit angrenzt. Das Projizieren der Positionsinformation des Objekts erfolgt bevorzugt automatisch und/oder selbsttätig mittels der Projektionseinheit.

Die vorliegende Erfindung kann vorteilhaft für alle, dem Fachmann als sinnvoll erscheinenden medizinischen Bildgebungsuntersuchungen eingesetzt werden, wie beispielweise für eine Computertomographieuntersuchung, eine PET-Untersuchung (Positron-Emissions-Tomographie-Untersuchung, usw. Besonders vorteilhaft jedoch eignet sich die vorliegend Erfindung für eine Magnetresonanzuntersuchung, da hier zur Vorbereitung des Patienten stets lokale Hochfrequenzantenneneinheiten am Patienten angeordnet werden.

Die Erfindung ermöglicht eine vorteilhafte Unterstützung eines medizinischen Bedienpersonals während einer Vorbereitung eines Patienten für eine medizinische Bildgebungsuntersuchung. Insbesondere kann derart für ein unerfahrenes und/oder ungeübtes medizinisches Bedienpersonal eine einfache und zeitsparende Hilfestellung zur Positionierung des Patienten und/oder zur Anordnung und/oder Positionierung von Zubehöreinheiten bereitgestellt werden. Folglich kann durch die Unterstützung für eine optimierte Lagerung des Patienten und/oder der Zubehöreinheit eine Fehlpositionierung verhindert werden und damit eine Bildqualität vorteilhaft erhöht werden. Hierdurch können auch Messwiederholungen aufgrund von Fehlpositionierungen vorteilhaft verhindert werden.

In einer vorteilhaften Weiterbildung der Erfindung kann es vorgesehen sein, dass die Erfassungseinheit eine Kamera umfasst. Die Erfassungseinheit umfasst bevorzugt eine 2D-Kamera und/oder eine 3D-Kamera. Hierdurch kann eine besonders kostengünstige Erfassungseinheit zur Erfassung der Patientendaten bereitgestellt werden.

Alternativ oder zusätzlich kann die Erfassungseinheit auch eine Sensoreinheit umfassen, welche Sensoreinheit die Patientendaten erfasst. Dabei kann die Sensoreinheit innerhalb der Patientenlagerungsvorrichtung integriert sein, wie beispielsweise Drucksensoren und/oder Hochfrequenzsensoren umfassen, welche Sensoren innerhalb einer Liegefläche der Patientenlagerungsvorrichtung integriert sind, so dass anhand einer Druckverteilung auf eine Lage und/oder Orientierung und/oder Größe Und/oder Position des Patienten geschlossen werden kann.

In einer vorteilhaften Weiterbildung der Erfindung kann es vorgesehen sein, dass die Patientendaten Positionsdaten des Patienten umfassen. Die Positionsdaten können dabei eine Position des auf der Patientenlagerungsvorrichtung positionierten Patienten umfassen. Zudem können die Positionsdaten auch eine Abmessung eines stehenden Patienten umfassen. Die Positionsdaten können aber auch eine Position des zu untersuchenden Bereichs des Patienten umfassen. Die Positionsdaten können dabei sowohl eine Position des Patienten, insbesondere des zu untersuchenden Bereichs des Patienten, bezüglich eines Referenzpunktes und/oder einer Referenzposition auf der Patientenlagerungsvorrichtung umfassen, als auch eine Ausdehnung des Patienten, wie insbesondere eine Länge und/oder eine Größe und/oder eine Breite und/oder eine Höhe des Patienten.

Durch diese Ausgestaltung der Erfindung kann eine optimale Position des Patienten für die anstehende medizinische Bildgebungsuntersuchung erreicht werden. Zudem kann auch eine Position einer Zubehöreinheit an die Position und/oder an eine körperliche Ausdehnung des Patienten angepasst werden. Insbesondere kann derart das medizinische Bedienpersonal eine patientenangepasste oder eine patientenindividuelle Unterstützung zur Vorbereitung des Patienten erhalten und somit eine zeitsparende Lagerung des Patienten und/oder der Zubehöreinheit an dem Patienten ermöglicht werden.

In einer vorteilhaften Weiterbildung der Erfindung kann es vorgesehen sein, dass für die Erfassung der Patientendaten Landmarken am Patienten angeordnet sind. Hierdurch kann eine eindeutige Identifizierung einzelner Körperbereich des Patienten und/oder des zu untersuchenden Bereichs des Patienten mittels der Erfassungseinheit und/oder der Berechnungseinheit erfolgen. Insbesondere kann derart auch eindeutig eine Orientierung des Patienten und/oder des zu untersuchenden Bereichs ermittelt werden.

In einer vorteilhaften Weiterbildung der Erfindung kann es vorgesehen sein, dass die Daten der Zubehöreinheiten in einer Datenbank hinterlegt sind. Diese Ausgestaltung der Erfindung ermöglicht einen schnellen und/oder direkten Zugriff auf die Daten der Zubehöreinheiten. Insbesondere können derart der Berechnungseinheit alle zur Verfügung stehenden Zubehöreinheiten für die Bestimmung und/oder Berechnung der Positionsinformation des Objekts in einfacher Art und Weise zur Verfügung stehen. Die Datenbank kann dabei innerhalb der medizinischen Bildgebungsvorrichtung, wie beispielsweise einer Speichereinheit der medizinischen Bildgebungsvorrichtung, integriert sein. Zudem ist es denkbar, dass die Datenbank in einer zentralen Speichereinheit eines Betreibers der medizinischen Bildgebungsvorrichtung integriert ist. Des Weiteren kann es auch vorgesehen sein, dass die Datenbank von einer externen Einheit, wie beispielsweise einer Cloud, umfasst ist.

In einer vorteilhaften Weiterbildung der Erfindung kann es vorgesehen sein, dass anhand der erfassten Patientendaten ein Körpermodell bestimmt wird. Hierdurch kann vorteilhaft eine Lage und/oder Position von Organen und/oder weiteren zu untersuchenden Bereichen, wie beispielweise Gelenke usw., schnell und zuverlässig bestimmt und/oder erkannt werden. Dies ermöglicht auch eine einfache Bestimmung einer optimalen Position des Organs und/oder des zu untersuchenden Bereichs des Patienten für die medizinische Bildgebungsuntersuchung. Dies ermöglicht auch eine korrekte Positionierung von Zubehöreinheiten bezüglich des zu untersuchenden Bereichs, so dass hierdurch vorteilhaft eine Bildqualität erhöht werden kann.

Das Körpermodell wird mittels der Berechnungseinheit bestimmt, wobei hierzu die Berechnungseinheit einen Körpermodell-Algorithmus aufweist. Vorzugsweise erfolgt die Berechnung und/oder Bestimmung des Körpermodells anhand der erfassten Patientendaten.

In einer vorteilhaften Weiterbildung der Erfindung kann es vorgesehen sein, dass in dem Schritt des Berechnens des Positionsinformation des Objekts anhand der Untersuchungsinformation und/oder anhand der Patientendaten zumindest eine Zubehöreinheit von mehreren zur Verfügung stehenden Zubehöreinheiten ausgewählt wird. Insbesondere kann derart eine korrekte Auswahl der Zubehöreinheit für die anstehende medizinische Bildgebungsuntersuchung für ein ungeübtes und/oder unerfahrenes medizinisches Bedienpersonal vereinfacht werden. Vorzugsweise erfolgt die Auswahl der zumindest einen Zubehöreinheit aus den mehreren zur Verfügung stehenden Zubehöreinheiten automatisch und/oder selbsttätig mittels der Berechnungseinheit.

Beispielsweise kann bei einer Erfassung einer "Feed-First"-Position des Patienten, in der der Patient mit den Füssen voran in den Patientenaufnahmebereich für die medizinische Bildgebungsuntersuchung gefahren wird, zunächst nur Zubehöreinheiten ausgewählt werden, die für Fußuntersuchungen und/oder Beinuntersuchungen vorgesehen sind, wie insbesondere eine Fuß-Hochfrequenzantenneneinheit oder eine Knie-Hochfrequenzantenneneinheit. Beispielsweise kann bei einer Erfassung einer "Head-First"-Position des Patienten, in der der Patient mit dem Kopf voran in den Patientenaufnahmebereich für die medizinische Bildgebungsuntersuchung gefahren wird, zunächst nur Zubehöreinheiten ausgewählt werden, die für Kopfuntersuchungen und/oder Brustuntersuchungen vorgesehen sind, wie insbesondere eine Kopf-Hochfrequenzantenneneinheit und/oder Brust-Hochfrequenzantenneneinheit usw. Zudem kann auch die Auswahl der Zubehöreinheit anhand einer Patientengröße erfolgen. Beispielsweise kann auch anhand einer Größe des zu untersuchenden Körperbereichs des Patienten eine Auswahl vorgegeben werden. So kann bei Patienten, deren Kopfumfang derart groß ist, dass ein zur Verfügung stehender Kopfaufnahmebereich einzelner Kopf-Hochfrequenzantenneneinheiten zu klein ist für die Aufnahme des Kopfes des Patienten oder des Kopfes zusammen mit Kopfhörern, eine entsprechende Auswahl an großen Kopf-Hochfrequenzantenneneinheit dem medizinischen Bedienpersonal angezeigt werden. Zudem kann für kleine Patienten eine kleinere Körper-Hochfrequenzantenneneinheit ausgewählt werden als für große Patienten. Anhand der Untersuchungsinformation kann zudem der zu untersuchende Bereich weiter spezifiziert werden, so dass konkret eine Zubehöreinheit ausgewählt werden kann. Die Untersuchungsinformation kann beispielsweise eine Untersuchungsregion und/oder einen zu untersuchenden Bereich und/oder ein Krankheitsbild des Patienten umfassen.

Die mehreren zur Verfügung stehenden Zubehöreinheiten umfassen bevorzugt alle vor Ort, also am Ort der medizinischen Bildgebungsvorrichtung, für medizinische Bildgebungsuntersuchungen zur Verfügung stehende Zubehöreinheiten. Dabei kann es auch vorgesehen sein, dass die Erfassungseinheit auch die einzelnen Zubehöreinheiten erfassen kann, beispielsweise indem der Erfassungsbereich der Erfassungseinheit auf ein Regal, das die Zubehöreinheiten beinhaltet, gerichtet ist. Dabei kann auch die Projektionseinheit beispielsweise die ausgewählte Zubehöreinheit ausleuchten und/oder anleuchten, so dass eine einfache Auswahl für das medizinische Bedienpersonal erfolgen kann.

In einer vorteilhaften Weiterbildung der Erfindung kann es vorgesehen sein, dass die Positionsinformation des Objekts eine Sollposition des Patienten umfasst, welche Sollposition des Patienten auf die Patientenlagerungsvorrichtung und/oder dem Patienten projiziert wird. Die Sollposition des Patienten umfasst bevorzugt eine ideale Position des Patienten für die anstehende medizinische Bildgebungsuntersuchung, wobei der Patient die Sollposition für die medizinische Bildgebungsuntersuchung einnehmen sollte, um eine hohe Bildqualität in den erfassten Bilddaten zu erhalten. Insbesondere umfasst die Sollposition des Patienten eine optimale Ausrichtung des zu untersuchenden Bereichs bezüglich der Patientenlagerungsvorrichtung und/oder bezüglich von Zubehöreinheiten, wie beispielsweise einer Kopf-Hochfrequenzantenneneinheit und/oder einer Knie-Hochfrequenzantenneneinheit. Derartige Zubehöreinheiten können eine vorgegebene Position bezüglich der Patientenlagerungsvorrichtung einnehmen, so dass eine Lagerung des Patienten an diese Position angepasst werden muss.

Alternativ oder zusätzlich kann die Positionsinformation auch eine Positionsänderung des Patienten umfassen, welche Positionsänderung auf die Patientenlagerungsvorrichtung und/oder den Patienten projiziert wird. Eine Positionsänderung kann beispielsweise von der Berechnungseinheit anhand einer Abweichung der Sollposition des Patienten von einer aktuellen Position des Patienten ermittelt werden, wobei die aktuelle Position des Patienten anhand der erfassten Patientendaten ermittelt werden kann. Die Positionsänderung kann beispielsweise durch Pfeile, die auf dem Patienten und/oder der Patientenlagerungsvorrichtung projiziert werden, angezeigt werden. Durch die Pfeile kann eine Richtung für das medizinische Bedienpersonal angezeigt werden, in welche Richtung der Patienten verschoben werden soll. Zudem kann die Positionsänderung auch durch eine Projektion einer Kontur des Patienten an der gewünschten Sollposition erfolgen. Dabei können auch nur einzelnen Körperteile und/oder Körperbereich des Patienten von einer Positionsänderung betroffen sein. So sollten beispielsweise bei Leberuntersuchungen von Patienten die Arme des Patienten nicht zu nah am Patienten anliegen, um Artefakte in den Bilddaten zu vermeiden. Eine derartige Sollposition der Arme des Patienten kann mittels der Projektionseinheit auf die Patientenlagerungsvorrichtung projiziert werden.

In einer vorteilhaften Weiterbildung der Erfindung kann es vorgesehen sein, dass die Positionsinformation eine Sollposition der Zubehöreinheit umfasst, welche Sollposition auf dem Patienten projiziert wird. Dies ermöglicht eine besonders einfache und zeitsparende Anbringung und/oder Positionierung der Zubehöreinheit auch für ein ungeübtes und/oder unerfahrenes medizinisches Bedienpersonal. Zubehöreinheiten, wie beispielsweise Hochfrequenzantenneneinheiten mit einem Pilot-Tone-Transmitter sollten derart positioniert werden, dass der Pilot-Tone-Transmitter möglichst nahe am Herzen des Patienten angeordnet ist. Hierbei kann insbesondere eine Sollposition des Pilot-Tone-Transmitters auf dem Patienten projiziert werden für eine einfache Positionierung der entsprechenden Hochfrequenzantenneneinheit.

In einer vorteilhaften Weiterbildung der Erfindung kann es vorgesehen sein, dass nach dem Projizieren der Positionsinformation des Objekts ein Positionieren des Objekts, auf das sich die Positionsinformation bezieht, erfolgt und anschließend Objektdaten mittels der Erfassungseinheit erfasst werden und mit der Sollposition des Objekts verglichen werden. Vorzugsweise erfolgt das Positionieren des Objekts manuell durch das medizinische Bedienpersonal. Anhand der erfassten Objektdaten kann kontinuierlich eine Position des Objekts während der Vorbereitung des Patienten überwacht und/oder kontrolliert werden. Zudem kann derart auch dem medizinischen Bedienpersonal jede gewünschte Lageänderung und/oder Positionsänderung des Objekts unmittelbar mitgeteilt werden. Die Sollposition des Objekts kann dabei eine Sollposition des Patienten und/oder auch eine Sollposition der Zubehöreinheit umfassen.

In einer vorteilhaften Weiterbildung der Erfindung kann es vorgesehen sein, dass ein Bestätigungssignal ausgegeben wird, sofern die erfassten Objektdaten mit der Sollposition übereinstimmen. Das Bestätigungssignal kann dabei ein optisches und/oder ein akustisches Bestätigungssignal umfassen, wie beispielsweise ein Ausgabe eines Tons und/oder einer Tonfolge und/oder eine Anzeige eines Farbwechsels an der Scannereinheit und/oder Ausgabe eines blinkenden Lichts usw. Hierdurch kann das medizinische Bedienpersonal ein direktes Feedback für einzelne Vorbereitungsschritte während der Vorbereitung des Patienten erhalten.

In einer vorteilhaften Weiterbildung der Erfindung kann es vorgesehen sein, dass erneut die Positionsinformation projiziert wird, sofern die erfassten Objektdaten mit der Sollposition des Objekts divergieren. Hierdurch kann das medizinische Bedienpersonal ein direktes Feedback für einzelne Vorbereitungsschritte während der Vorbereitung des Patienten erhalten.

Des Weiteren geht die Erfindung aus von einer medizinischen Bildgebungsvorrichtung, die zu einer Ausführung eines Verfahrens zur Vorbereitung eines Patienten für eine medizinische Bildgebungsuntersuchung nach einem der vorhergehenden Ansprüche ausgelegt ist, wobei die medizinische Bildgebungsvorrichtung eine Scannereinheit, eine Patientenlagerungsvorrichtung zur Lagerung des Patienten für die medizinische Bildgebungsuntersuchung, eine Erfassungseinheit zur Erfassung von Patientendaten, eine Berechnungseinheit zur Berechnung von Positionsdaten und eine Projektionseinheit zur Projektion der Positionsdaten aufweist.

Hierdurch kann eine vorteilhafte Unterstützung eines medizinischen Bedienpersonals während einer Vorbereitung eines Patienten für eine medizinische Bildgebungsuntersuchung bereitgestellt werden. Insbesondere kann derart für ein unerfahrenes und/oder ungeübtes medizinisches Bedienpersonal eine einfache und zeitsparende Hilfestellung zur Positionierung des Patienten und/oder zur Anordnung und/oder Positionierung von Zubehöreinheiten bereitgestellt werden. Folglich kann durch die Unterstützung für eine optimierte Lagerung des Patienten und/oder der Zubehöreinheit eine Fehlpositionierung verhindert werden und damit eine Bildqualität vorteilhaft erhöht werden. Hierdurch können auch Messwiederholungen aufgrund von Fehlpositionierungen vorteilhaft verhindert werden.

Die Vorteile der erfindungsgemäßen medizinischen Bildgebungsvorrichtung entsprechen im Wesentlichen den Vorteilen der erfindungsgemäßen medizinischen Sicherungseinheit, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Des Weiteren geht die Erfindung aus von einem Computerprogrammprodukt, welches ein Programm umfasst und direkt in einem Speicher einer programmierbaren Steuerungseinheit ladbar ist, mit Programmmitteln, um ein Verfahren zu einer Unterstützung einer Vorbereitung eines Patienten für eine medizinische Bildgebungsuntersuchung auszuführen, wenn das Programm in der Steuerungseinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch kompiliert und gebunden oder der nur interpretiert werden muss, oder einen ausführbaren Softwarecode umfassen, der zur Ausführung nur noch in eine entsprechende Recheneinheit zu laden ist.

Des Weiteren geht die Erfindung aus von einem computerlesbaren und/oder elektronisch lesbaren Datenträger, welcher ein Programm umfasst, das zu einer Ausführung eines Verfahrens zu einer Unterstützung einer Vorbereitung eines Patienten für eine medizinische Bildgebungsuntersuchung vorgesehen ist.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße medizinische Bildgebungsvorrichtung in einer schematischen Darstellung,
- Fig. 2: ein erfindungsgemäßes Verfahren zu einer Unterstützung einer Vorbereitung eines Patienten für eine medizinische Bildgebungsuntersuchung und
- Fig. 3: ein Beispiel einer Projektion einer Positionsinformation auf dem Patienten.

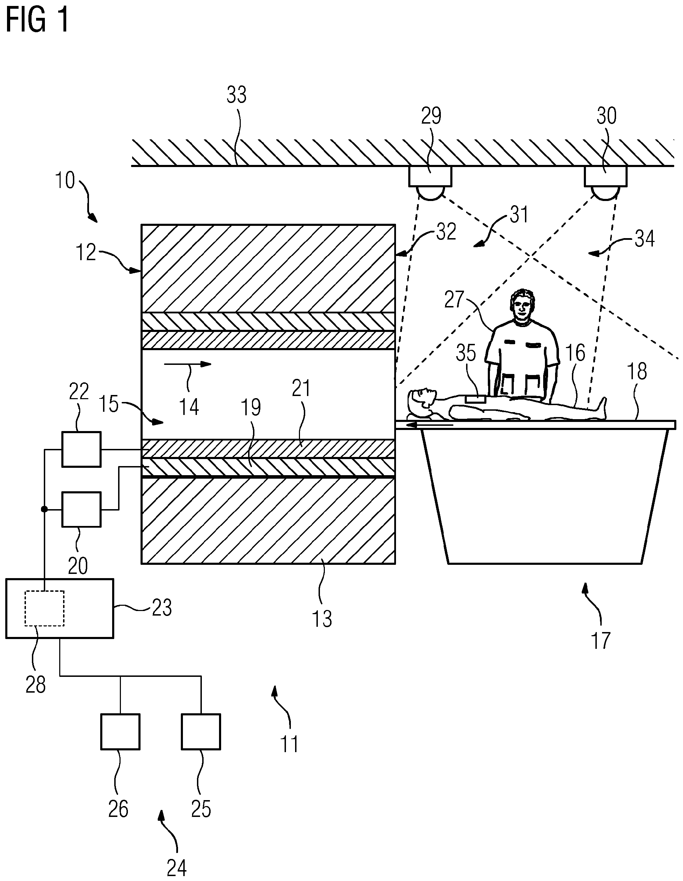

In der Figur 1 ist eine medizinische Bildgebungsvorrichtung 10 schematisch dargestellt. Die medizinische Bildgebungsvorrichtung 10 ist im vorliegenden Ausführungsbeispiel von einer Magnetresonanzvorrichtung 11 gebildet, wobei beispielshaft die vorliegende Erfindung anhand der Magnetresonanzvorrichtung 11 erläutert wird. Die vorliegende Erfindung ist jedoch nicht auf Ausgestaltung der medizinischen Bildgebungsvorrichtung 10 auf eine Magnetresonanzvorrichtung 11 beschränkt und weitere Ausgestaltungen der medizinischen Bildgebungsvorrichtung 10 sind jederzeit denkbar, wie beispielsweise eine Computertomographievorrichtung, eine PET-Vorrichtung usw.

Die Magnetresonanzvorrichtung 11 umfasst eine Scannereinheit 12, die einen supraleitenden Hauptmagneten 13 zu einem Erzeugen eines starken und insbesondere konstanten Hauptmagnetfelds 14 umfasst. Zudem weist die Magnetresonanzvorrichtung 11 einen Patientenaufnahmebereich 15 auf zu einer Aufnahme eines Patienten 16. Der Patientenaufnahmebereich 15 im vorliegenden Ausführungsbeispiel ist zylinderförmig ausgebildet und in einer Umfangsrichtung von der Scannereinheit 12 zylinderförmig umgeben. Grundsätzlich ist jedoch eine davon abweichende Ausbildung des Patientenaufnahmebereichs 15 jederzeit denkbar. Der Patient 16 kann mittels einer Patientenlagerungsvorrichtung 17 der Magnetresonanzvorrichtung 11 in den Patientenaufnahmebereich 15 geschoben und/oder gefahren werden. Die Patientenlagerungsvorrichtung 17 weist hierzu einen innerhalb des Patientenaufnahmebereichs 15 bewegbar ausgestalteten Patiententisch 18 auf.

Die Scannereinheit 12 weist weiterhin eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 20 der Magnetresonanzvorrichtung 10 gesteuert. Die Scannereinheit 11 umfasst weiterhin eine Hochfrequenzantenneneinheit 21 zu einer Anregung einer Polarisation, die sich in dem von dem Hauptmagneten 13 erzeugten Hauptmagnetfeld 14 einstellt. Die Hochfrequenzantenneneinheit 21 wird von einer Hochfrequenzantennensteuereinheit 22 der Magnetresonanzvorrichtung 11 gesteuert und strahlt hochfrequente Magnetresonanzsequenzen in einen Untersuchungsraum, der im Wesentlichen von einem Patientenaufnahmebereich 15 der Magnetresonanzvorrichtung 11 gebildet ist, ein.

Zu einer Steuerung des Hauptmagneten 13, der Gradientensteuereinheit 20 und zur Steuerung der Hochfrequenzantennensteuereinheit 22 weist die Magnetresonanzvorrichtung 11 eine Steuerungseinheit 23 auf. Die Steuerungseinheit 23 steuert zentral die Magnetresonanzvorrichtung 11, wie beispielsweise das Durchführen einer vorbestimmten bildgebenden Gradientenechosequenz. Zudem umfasst die Steuerungseinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 11 eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Anzeigeeinheit 25, beispielsweise auf zumindest einem Monitor, der Benutzerschnittstelle 24 für ein medizinisches Bedienpersonal 27 angezeigt werden. Weiterhin weist die Benutzerschnittstelle 24 eine Eingabeeinheit 26 auf, mittels der Informationen und/oder Parameter während eines Messvorgangs von dem medizinischen Bedienpersonal 27 eingegeben werden können.

Des Weiteren umfasst die Magnetresonanzvorrichtung 11 eine Berechnungseinheit 28, eine Erfassungseinheit 29 und eine Projektionseinheit 30. Mittels der Berechnungseinheit 28, der Erfassungseinheit 29 und der Projektionseinheit 30 kann ein Verfahren zu einer Unterstützung einer Vorbereitung eines Patienten 16 für eine medizinische Bildgebungsuntersuchung, im vorliegenden Ausführungsbeispiel für eine Magnetresonanzuntersuchung, ausgeführt werden. Das Verfahren zur Unterstützung einer Vorbereitung eines Patienten 16 für eine Magnetresonanzuntersuchung wird dabei automatisch und/oder selbsttätig mittels der Magnetresonanzvorrichtung 11, insbesondere der Berechnungseinheit 28 zusammen mit der Erfassungseinheit 29 und der Projektionseinheit 30 ausgeführt. Hierzu weist die Berechnungseinheit 28 einen nicht näher dargestellten Prozessor auf. Des Weiteren umfasst die Berechnungseinheit 28 auch entsprechende Berechnungsprogramme und/oder Berechnungssoftware. Die Berechnungsprogramme und/oder Berechnungssoftware können dabei innerhalb einer nicht näher dargestellten Speichereinheit der Berechnungseinheit 28 und/oder der medizinischen Bildgebungsvorrichtung 11 gespeichert sein. Zudem können die Berechnungsprogramme und/oder Berechnungssoftware auch in einem externen Speicher, wie beispielsweise einer Cloud, hinterlegt sein, wobei die Berechnungseinheit 28 hierbei mittels eines Datennetzwerks auf die Berechnungsprogramme und/oder Berechnungssoftware zugreifen kann.

Die Berechnungseinheit 28 kann innerhalb der Steuerungseinheit 23 integriert sein. Alternativ oder zusätzlich kann die Berechnungseinheit 28 auch als selbstständige Einheit ausgebildet sein.

Die Erfassungseinheit 29 ist im vorliegenden Ausführungsbeispiel von einer Kamera, insbesondere einer 2D-Kamera und/oder einer 3D-Kamera zur Erfassung von 2D-Patientendaten und/oder 3D-Patientendaten, gebildet. Die Erfassungseinheit 29 ist innerhalb eines Untersuchungsraums, innerhalb dessen auch die Scannereinheit 12 angeordnet und/oder installiert ist, angeordnet. Die Erfassungseinheit 29 ist dabei derart innerhalb des Untersuchungsraums angeordnet, dass ein Erfassungsbereich 31 der Erfassungseinheit 29, insbesondere der Kamera, einen Bereich, innerhalb dessen die Vorbereitung des Patienten 16 für die Magnetresonanzuntersuchung stattfindet, abdeckt. Dieser Erfassungsbereich 31 und/oder dieser Bereich befindet sich vorzugsweise vor der Scannereinheit 12 und dabei direkt angrenzend an eine Frontseite 32 der Scannereinheit 12. Der Erfassungsbereich 29 umfasst dabei auch einen Ort, in dem sich die Patientenlagerungsvorrichtung 17 für die Vorbereitung des Patienten 16 befindet.

Die Erfassungseinheit 29 kann dabei an der Scannereinheit 12, insbesondere an der Frontseite 32 der Scannereinheit 12, angeordnet sein. Besonders vorteilhaft ist die Erfassungseinheit 29 an einer Wand und/oder einer Raumdecke 33 des Untersuchungsraums angeordnet, so dass stets ein unverstellter Blick auf den Patienten 16 und/oder die Patientenlagerungsvorrichtung 17 während der Vorbereitung des Patienten 16 für die Magnetresonanzuntersuchung gewährleistet ist.

Alternativ hierzu kann die Erfassungseinheit 29 anstatt einer Kamera auch eine Sensoreinheit umfassen zur Erfassung der Patientendaten. Dabei kann die Sensoreinheit innerhalb der Patientenlagerungsvorrichtung 17 integriert sein, wie beispielsweise Drucksensoren, die innerhalb einer Liegefläche der Patientenlagerungsvorrichtung 17 integriert sind, so dass anhand einer Druckverteilung auf eine Lage und/oder Orientierung und/oder Größe Und/oder Position des Patienten 16 geschlossen werden kann. Zudem kann die Sensoreinheit auch Hochfrequenzsensoren zur Erfassung der Patientendaten aufweisen, die innerhalb der Patientenlagerungsvorrichtung 17, insbesondere innerhalb einer Lagerungsfläche der Patientenlagerungsvorrichtung 17 integriert sind.

Die Projektionseinheit 30 ist ebenfalls innerhalb des Untersuchungsraums, innerhalb dessen auch die Scannereinheit 12 angeordnet und/oder installiert ist, angeordnet. Die Projektionseinheit 30 ist dabei derart innerhalb des Untersuchungsraums angeordnet, dass ein Projektionsbereich 33 der Projektionseinheit 30 einen Bereich, innerhalb dessen die Vorbereitung des Patienten 16 für die Magnetresonanzuntersuchung stattfindet, abdeckt. Dieser Projektionsbereich 34 und/oder dieser Bereich befindet sich vorzugsweise vor der Scannereinheit 12 und dabei direkt angrenzend an die Frontseite 32 der Scannereinheit 12. Der Projektionsbereich 34 umfasst dabei auch einen Ort, in dem sich die Patientenlagerungsvorrichtung 17 für die Vorbereitung des Patienten 16 befindet.

Die Projektionseinheit 30 kann dabei an der Scannereinheit 12, insbesondere an der Frontseite 32 der Scannereinheit 12, angeordnet sein. Besonders vorteilhaft ist die Projektionseinheit 30 an der Wand und/oder der Raumdecke 33 des Untersuchungsraums angeordnet, so dass stets ein unverstellter Blick auf den Patienten 16 und/oder die Patientenlagerungsvorrichtung 17 während der Vorbereitung des Patienten 16 für die Magnetresonanzuntersuchung gewährleistet ist.

Alternativ oder zusätzlich kann die Projektionseinheit 30 auch eine LED-Leiste an der Patientenlagerungsvorrichtung 17, insbesondere am Patiententisch 18 aufweisen, wobei mittels der LED-Leiste ein Bereich des Patienten 16 markiert werden kann, der mit einer Zubehöreinheit 35, beispielsweise mit einer lokalen Hochfrequenzantenneneinheit, versehen werden soll. Beispielsweise kann hierbei die LED-Leiste einen Bereich ausleuchten, der ein Knie des Patienten 16 umfasst, so dass für das medizinische Bedienpersonal 27 die erforderliche Knie-Hochfrequenzantenneneinheit ausgewählt werden kann.

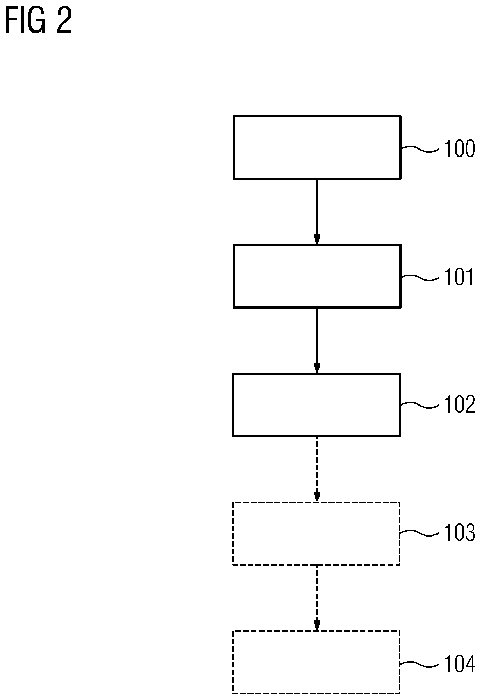

Das Verfahren zu einer Unterstützung eines medizinischen Bedienpersonals 27 während einer Vorbereitung eines Patienten 16 für eine medizinische Bildgebungsuntersuchung, insbesondere eine Magnetresonanzvorrichtung, ist in Fig. 2 dargestellt. Zu Beginn des Verfahrens befindet sich der Patient 16 bereits neben der Patientenlagerungsvorrichtung 17 oder ist bereits auf der Patientenlagerungsvorrichtung 17 positioniert. In einem ersten Verfahrensschritt 100 erfolgt ein Erfassen von Patientendaten eines neben der Patientenlagerungsvorrichtung 17 befindlichen Patienten 16 oder eines auf der Patientenlagerungsvorrichtung 17 positionierten Patienten 16 mittels der Erfassungseinheit 29.

Die erfassten Patientendaten umfassen bevorzugt Positionsdaten des Patienten 16. Sofern der Patient 16 bereits auf Patientenlagerungsvorrichtung 17 positioniert ist, können die Positionsdaten des Patienten 16 dabei eine Position und/oder eine Lage und/oder eine Orientierung des Patienten 16 bezüglich der Patientenlagerungsvorrichtung 17 umfassen. Des Weiteren können die Positionsdaten auch eine Ausdehnung des Patienten 16, insbesondere eine Größe und/oder eine Länge und/oder eine Breite und/oder eine Höhe, umfassen.

Zur einfacheren Erfassung der Positionsdaten kann zudem der Patient 16 mit Landmarkern versehen sein. Bevorzugt ist hierbei auch ein zu untersuchender Bereich des Patienten 16 mit Landmarkern versehen.

Anschließend werden die erfassten Patientendaten von der Erfassungseinheit 29 an die Berechnungseinheit 28 mittels einer nicht näher dargestellten Datenübertragungseinheit der Magnetresonanzvorrichtung 11 übertragen.

In einem weiteren Verfahrensschritt 101 erfolge ein Berechnen einer Positionsinformation eines Objekts mittels der Berechnungseinheit 28. Das Berechnen erfolgt hierbei anhand der Patientendaten und/oder anhand einer Untersuchungsinformation und/oder anhand von Daten von Zubehöreinheiten 35. Die Daten der Zubehöreinheiten 35 sind dabei in einer nicht näher dargestellten Datenbank hinterlegt, wobei die Berechnungseinheit 28 mittels des Datennetzwerkes auf die Daten der Zubehöreinheiten 35 der Datenbank zugreifen kann. Die Daten der Zubehöreinheiten 35 umfassen typischerweise eine Geometrie, insbesondere eine Länge und/oder eine Breite und/oder eine Höhe, der einzelnen Zubehöreinheiten 35, die zusammen mit den jeweiligen Zubehöreinheiten 35 hinterlegt sind. Alternativ oder zusätzlich können die Daten der Zubehöreinheiten 35 auch eine Sollposition der einzelnen Zubehöreinheiten 35 bezüglich der Patientenlagerungsvorrichtung 17 und/oder bezüglich des Patienten 16 umfassen, wobei die Sollposition zusammen mit den jeweiligen Zubehöreinheiten 35 hinterlegt ist. Zudem können die Daten der einzelnen Zubehöreinheiten 35 auch einen Einsatzbereich, insbesondere für welche Art von Untersuchungen die einzelnen Zubehöreinheiten 35 verwendet werden können, umfassen, der zusammen mit den jeweiligen Zubehöreinheiten 35 hinterlegt ist. Vorzugsweise sind innerhalb der Datenbank alle vor Ort zur Verfügung stehenden Zubehöreinheiten 35 hinterlegt.

Die einzelnen Zubehöreinheiten 35 können beispielsweise lokale Hochfrequenzantenneneinheiten, die für die Magnetresonanzuntersuchung um den zu untersuchenden Bereich des Patienten 16 positioniert werden, umfassen. Zudem können die Zubehöreinheiten 35 auch eine EKG-Einheit und/oder einen Atemsensor und/oder weitere, dem Fachmann als sinnvoll erscheinende Einheiten umfassen.

Anhand der erfassten Patientendaten kann zunächst ein Körpermodell des Patienten 16 mittels der Berechnungseinheit 28 erstellt und/oder berechnet werden. Vorzugsweise weist hierzu die Berechnungseinheit 28 einen Körpermodell-Algorithmus auf, mittels dessen das Körpermodell des Patienten anhand der erfassten Patientendaten ermittelt und/oder bestimmt werden kann.

Die Untersuchungsinformation kann dabei ein Krankheitsbild und/oder eine Untersuchungsregion des Patienten 16 umfassen. Die Untersuchungsinformation kann bereits innerhalb einer Patientendatenbank hinterlegt sein und somit von der Berechnungseinheit 28 selbsttätig abgerufen werden. Zudem kann es auch sein, dass die Untersuchungsinformation direkt von dem medizinischen Bedienpersonal 27 über die Eingabeeinheit 26 eingeben wird und anschließend der Berechnungseinheit 28 zur Verfügung steht.

Vorzugsweise wird von der Berechnungseinheit 28 anhand des Körpermodells und der Untersuchungsinformation und der Daten der Zubehöreinheiten 35 eine Positionsinformation eines Objekts bestimmt werden. Umfasst das Objekt den Patienten 16 kann dabei eine Position des Patienten 16 festgelegt oder überprüft werden. Beispielsweise kann zunächst eine Position des Patienten 16 für die anstehende medizinische Bildgebungsuntersuchung auf der Patientenlagerungsvorrichtung 17 festgelegt werden. Dabei kann die Position des Patienten 16 auch eine Ausrichtung des Patienten 16 auf der Patientenlagerungsvorrichtung 17 umfassen. Des Weiteren kann bei Kopfuntersuchungen oder Knieuntersuchungen des Patienten 16, bei denen die lokale Hochfrequenzantenneneinheit eine vorgegebene Position bezüglich der Patientenlagerungsvorrichtung 17 aufweisen, eine Sollposition des Patienten 16 bezüglich der Hochfrequenzantenneneinheit bestimmt und/oder berechnet werden. Zudem können für in die Patientenlagerungsvorrichtung 17 integrierte Zubehöreinheiten 35, wie beispielsweise integrierte Hochfrequenzantenneneinheiten mit integriertem Atemsensor und/oder EKG-Einheit, ebenfalls eine Sollposition des Patienten 16 bezüglich der Zubehöreinheit 35, insbesondere bezüglich der Hochfrequenzantenneneinheit und/oder des Atemsensors und/oder der EKG-Einheit, bestimmt und/oder berechnet werden.

Umfasst das Objekt dagegen eine Zubehöreinheit 35, kann somit in dem Verfahrensschritt 101 eine Positionsinformation, insbesondere eine Sollposition, der Zubehöreinheit 35 bestimmt und/oder berechnet werden. Beispielsweise kann hierbei eine Lage und/oder Orientierung und/oder Position einer als lokale Hochfrequenzantenneneinheit ausgebildete Zubehöreinheit 35 für die Magnetresonanzuntersuchung bestimmt werden. Zudem kann auch eine Lage und/oder Position einer als EKG-Elektroden ausgebildete Zubehöreinheit 35 bestimmt werden.

Zudem kann auch anhand der erfassten Patientendaten und anhand der Untersuchungsinformation in dem Verfahrensschritt 101 von der Berechnungseinheit 28 zumindest eine der mehreren zur Verfügung stehenden Zubehöreinheiten 35 ausgewählt werden. Beispielsweise kann eine als Körperspule ausgebildete Zubehöreinheit 35 anhand einer Größe des Patienten 16 ausgewählt werden.

In einem weiteren Verfahrensschritt 102 erfolgt ein Projizieren der Positionsinformation des Objekts mittels der Projektionseinheit 30. Umfasst hierbei die Positionsinformation eine Sollposition des Patienten 16, so wird die Sollposition des Patienten 16 auf die Patientenlagerungsvorrichtung 17 und/oder dem Patienten 16 projiziert. Die Sollposition kann dabei durch eine ausgeleuchtete Fläche, die die Sollposition des Patienten 16 markiert, und/oder Umriss und/oder einen Umfang der Sollposition umfassen.

Zudem kann die Sollposition auch eine Positionsänderung des Patienten 16 umfassen, die auf dem Patienten 16 und/oder der Patientenlagerungsvorrichtung 17 projiziert wird. Die Positionsänderung kann beispielsweise mittels projizierter Pfeile angezeigt werden.

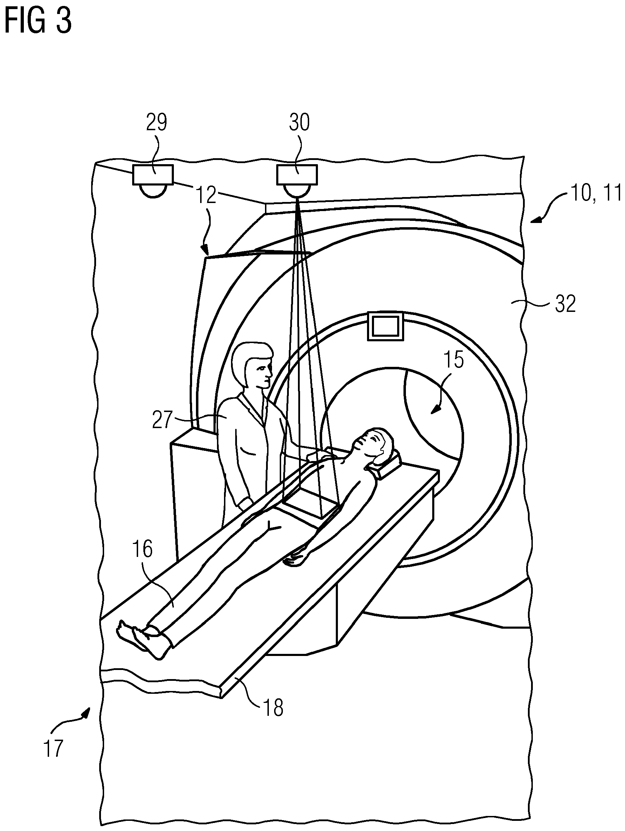

Umfasst die Positionsinformation des Objekts eine Sollposition einer Zubehöreinheit 35, kann diese Sollposition der Zubehöreinheit 35 auch auf dem Patienten 16 projiziert werden. Beispielsweise kann anhand einer projizierten Fläche und/oder eines projizierten Umfangs die Sollposition der Zubehöreinheit 35 auf dem Patienten 16 angezeigt werden. In Fig. 3 ist beispielhaft eine Projektion einer Sollposition einer Zubehöreinheit 35 auf dem auf der Patientenlagerungsvorrichtung 17 positionierten Patienten 16 dargestellt.

Gleichzeitig mit der Projektion der Sollposition des Objekts kann zusätzlich auch eine Ausgabe über ein Display an der Scannereinheit 12 erfolgen, wie beispielsweise eine Ausgabe in textueller Form.

In einem weiteren optionalen Verfahrensschritt 103 erfolgt nach dem Projizieren der Positionsinformation des Objekts ein Positionieren des Objekts. Beispielsweise wird hierbei von dem medizinischen Bedienpersonal 27 die Zubehöreinheit 35 in der angezeigten und/oder projizierten Sollposition positioniert. Alternativ oder zusätzlich kann auch Patient 16 in der angezeigten und/oder projizierten Sollposition von dem medizinischen Bedienpersonal 27 positioniert werden.

Anschließend erfolgt ebenfalls in einem optionalen weiteren Verfahrensschritt 104 ein Erfassen von Objektdaten mittels der Erfassungseinheit 29, wobei von der Berechnungseinheit 28 die erfassten Objektdaten mit der Sollposition des Objekts verglichen werden. Zur einfacheren Erfassung von Objektdaten, insbesondere von Objektdaten einer Zubehöreinheit 35, kann diese auch zur Erkennung mittels Markern versehen sein. Stimmen die erfassten Objektdaten mit der Sollposition des Objekts überein, erfolgt eine Ausgabe eines Bestätigungssignals, beispielsweise eines akustischen und/oder optischen Bestätigungssignals. Divergieren dagegen die erfassten Objektdaten mit der Sollposition des Objekts, wird erneut die Positionsinformation, insbesondere die Sollposition des Objekts, mittels der Projektionseinheit 30 projiziert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer Unterstützung einer Vorbereitung eines Patienten für eine medizinische Bildgebungsuntersuchung, insbesondere eine Magnetresonanzuntersuchung, mit den folgenden Schritten:
- Erfassen von Patientendaten eines Patienten mittels einer Erfassungseinheit,
- Berechnen einer Positionsinformation eines Objekts mittels einer Berechnungseinheit, wobei das Berechnen der Positionsinformation des Objekts anhand der Patientendaten und/oder anhand einer Untersuchungsinformation und/oder anhand von Daten von Zubehöreinheiten erfolgt, und
- Projizieren der Positionsinformation des Objekts mittels einer Projektionseinheit.

2. Verfahren nach einem der vorhergehenden Ansprüche, **dadurch gekennzeichnet, dass** die Erfassungseinheit eine Kamera umfasst.

3. Verfahren nach einem der vorhergehenden Ansprüche, **dadurch gekennzeichnet, dass** die Patientendaten Positionsdaten des Patienten umfassen.

4. Verfahren nach einem der vorhergehenden Ansprüche, **dadurch gekennzeichnet, dass** für die Erfassung der Patientendaten Landmarken am Patienten angeordnet sind.

5. Verfahren nach einem der vorhergehenden Ansprüche, **dadurch gekennzeichnet, dass** die Daten der Zubehöreinheiten in einer Datenbank hinterlegt sind.

6. Verfahren nach einem der vorhergehenden Ansprüche, **dadurch gekennzeichnet, dass** anhand der erfassten Patientendaten ein Körpermodell bestimmt wird.

7. Verfahren nach einem der vorhergehenden Ansprüche, **dadurch gekennzeichnet, dass** in dem Schritt des Berechnens der Positionsinformation des Objekts anhand der Untersuchungsinformation und/oder anhand der Patientendaten zumindest eine Zubehöreinheit von mehreren zur Verfügung stehenden Zubehöreinheiten ausgewählt wird.

8. Verfahren nach einem der vorhergehenden Ansprüche, **dadurch gekennzeichnet, dass** die Positionsinformation des Objekts eine Sollposition des Patienten umfasst, welche Sollposition des Patienten auf die Patientenlagerungsvorrichtung und/oder dem Patienten projiziert wird.

9. Verfahren nach einem der vorhergehenden Ansprüche, **dadurch gekennzeichnet, dass** die Positionsinformation eine Positionsänderung des Patienten umfasst, welche Positionsänderung auf die Patientenlagerungsvorrichtung und/oder den Patienten projiziert wird.

10. Verfahren nach einem der vorhergehenden Ansprüche, **dadurch gekennzeichnet, dass** die Positionsinformation eine Sollposition der Zubehöreinheit umfasst, welche Sollposition auf dem Patienten projiziert wird.

11. Verfahren nach einem der vorhergehenden Ansprüche, **dadurch gekennzeichnet, dass** nach dem Projizieren der Positionsinformation des Objekts ein Positionieren des Objekts, auf das sich die Positionsinformation bezieht, erfolgt und anschließend Objektdaten mittels der Erfassungseinheit erfasst werden und mit der Sollposition des Objektes verglichen werden.

12. Verfahren nach einem der vorhergehenden Ansprüche, **dadurch gekennzeichnet, dass** ein Bestätigungssignal ausgegeben wird, sofern die erfassten Objektdaten mit der Sollposition des Objekts übereinstimmen.

13. Verfahren nach einem der vorhergehenden Ansprüche, **dadurch gekennzeichnet, dass** erneut die Positionsinformation projiziert wird, sofern die erfassten Objektdaten mit der Sollposition des Objekts divergieren.

14. Medizinische Bildgebungsvorrichtung, die zur Ausführung eines Verfahrens zur Vorbereitung eines Patienten für eine medizinische Bildgebungsuntersuchung nach einem der vorhergehenden Ansprüche ausgelegt ist, wobei die medizinische Bildgebungsvorrichtung eine Scannereinheit, eine Patientenlagerungsvorrichtung zur Lagerung des Patienten für die medizinische Bildgebungsuntersuchung, eine Erfassungseinheit zur Erfassung von Patientendaten, eine Berechnungseinheit zur Berechnung von Positionsdaten und eine Projektionseinheit zur Projektion der Positionsdaten aufweist.

15. Computerprogrammprodukt, welches ein Programm umfasst und direkt in einen Speicher einer programmierbaren Steuerungseinheit ladbar ist, mit Programmmitteln, um ein Verfahren zu einer Unterstützung einer Vorbereitung eines Patienten für eine medizinische Bildgebungsuntersuchung nach einem der Ansprüche 1 bis 13 auszuführen, wenn das Programm in der Steuerungseinheit ausgeführt wird.

16. Elektronisch lesbarer Datenträger mit darauf gespeicherten elektronisch lesbaren Steuerinformationen, welche derart ausgestaltet sind, dass sie bei Verwendung des Datenträgers in einer Steuerungseinheit das Verfahren zu einer Unterstützung einer Vorbereitung eines Patienten für eine medizinische Bildgebungsuntersuchung nach einem der Ansprüche 1 bis 13 durchführen.
